# EUROPÄISCHE PATENTSCHRIFT

(11) **EP 2 709 688 B1**
(45) Veröffentlichungstag und Bekanntmachung des Hinweises auf die Patenterteilung: **03.01.2018**
(21) Anmeldenummer: 12720910.4
(22) Anmeldetag: 16.05.2012
(51) Int. Cl.: B01F 13/08, F16C 32/04, H02K 7/09, H02K 29/08, A61M 1/10

(54) **ROTATIONSMASCHINE, SOWIE VORRICHTUNG MIT EINER ROTATIONSMASCHINE**
ROTATION MACHINE AND DEVICE WITH THE SAME
MACHINE ROTATIVE ET DISPOSITIF DOTÉ D'UNE MACHINE ROTATIF

(30) Priorität: 20.05.2011 EP 11166923
(43) Veröffentlichungstag der Anmeldung: 26.03.2014
(73) Patentinhaber: Levitronix GmbH, 8005 Zürich (CH)
(72) Erfinder: SCHÖB, Reto, CH-8964 Rudolfstetten (CH)
(74) Vertreter: Intellectual Property Services GmbH
(86) Internationale Anmeldenummer: PCT/EP2012/059169
(87) Internationale Veröffentlichungsnummer: WO 2012/159966

(56) Entgegenhaltungen:
- EP-A1- 0 900 572
- WO-A2-2011/024470
- US-A- 5 863 179
- US-A1- 2005 151 437
- US-A1- 2009 121 571

## Beschreibung

Die Erfindung betrifft eine als lagerloser Motor ausgestaltete Rotationsmaschine, einen Rotor für eine solche Rotationsmaschine, sowie eine Vorrichtung mit einer als lagerlosem Motor ausgestalteten Rotationsmaschine , insbesondere Wafer Bearbeitungsanlage, Bloreaktoranlage, Pumpe oder Mischer oder eine andere
Bearbeitungsanlage mit einer Rotationsmaschine gemäss dem Oberbegriff des unabhängigen Anspruchs 1. Rotationsmaschinen, die als sogenannter lagerloser Motor ausgestaltet sind, sind als Drehantrieb für verschiedenste Einrichtungen und Anwendungen seit langem im Stand der Technik etabliert. Solche lagerlosen Motoren haben einen als Lager- und Antriebsstator ausgestalteten Stator und einen innerhalb des Stators magnetisch berührungslos gelagerten magnetischen Rotor und kommen wegen ihrer besonderen Vorteile, nämlich vor allem wegen ihrer stabilen Laufeigenschaften und dem Fehlen eines jeglichen mechanischen Lagers am Rotor, vor allem in vielen Spezielanwendungen als Pumpen oder Dosierelnrichtungen, z.B, in der Halbleiterindustrie zur Handhabung von mechanisch aggressiven Flüssigkeiten wie Slurry immer mehr zum Einsatz. Unter anderem ist ein solcher Drehantrieb In der EP 1 063 753 beschrieben.
Die WO 2011/024470 A2 offenbart ein magnetisches Lager, das von einem separaten externen elektrischen Antrieb von aussen angetrieben wird und darf insofern nicht mit der vorliegenden Erfindung verwechselt werden.

Ein anderes klassisches Anwendungsgebiet solcher lagerloser Motoren ist die Behandlung empfindlicher oder hochreiner Flüssigkeiten. So wird die Technik der lagerlosen Motoren zum Beispiel sehr vorteilhaft in Blutpumpen oder in Bioreaktoren verwendet, oder zur Handhabung hochempfindlicher bzw. hochreiner Flüssigkeiten in der pharmazeutischen und chemischen Industrie, sowie allgemein in der Medizin bzw. der medizinischen Technik eingesetzt.

Die Krafterzeugung zur magnetischen Lagerung bekannter lagerloser Motoren in Scheibenläufer Ausführung erfolgt dabei nur in einer Ebene, nämlich in der Ebene des Rotors. Der Rotor wird in dieser Ebene in beide Radialrichtungen aktiv stabilisiert, wobei die Stabilisierung in Axialrichtung und die Stabilisierung der Verkippungsneigung bei den bekannten Motoren rein passiv durch Reluktanzkräfte erfolgt.

Ein bekannter lagerloser Motor, der unter anderen durch die vorliegende Erfindung verbessert werden soll, wird beispielsweise in der WO-A-96/31934 oder in einer anderen Variante auch in der EP-A-0 900 572 offenbart und das Prinzip solcher lagerlosen Motoren, die die vorliegende Erfindung unter anderem betrifft, soll im folgenden zum besseren Verständnis etwas näher erläutert werden.

Mit dem Begriff lagerloser Motor ist im Rahmen dieser Anmeldung gemeint, dass der Rotor vollkommen magnetisch gelagert ist, wobei keine separaten mechanischen Lager vorgesehen sind. Der Stator ist dazu als Lager- und Antriebsstator ausgestaltet; er ist also sowohl Stator des elektrischen Antriebs als auch Stator der magnetischen Lagerung. Dazu umfasst die Wicklung des Stators eine Antriebswicklung und eine Steuerwicklung, die prinzipiell irgendwo am Stator, im Speziellen auf ein und demselben Statorzahn oder in anderen Beispielen auch auf getrennten Statorzähnen vorgesehen sein können, wobei insbesondere bei modernen lagerlosen Motoren auch eine einzige elektrische Wicklung gleichzeitig als Antriebs- und Steuerwicklung dienen kann. Mit den elektrischen Wicklungen lässt sich ein magnetisches Drehfeld erzeugen, welches zum einen ein Drehmoment auf den Rotor ausübt, das dessen Rotation bewirkt, und welches zum anderen eine beliebig einstellbare Querkraft auf den Rotor ausübt, sodass dessen radiale Position aktiv steuerbar bzw. regelbar ist. Nur wenn der Rotor als scheibenförmiger oder ringförmiger Rotor ausgebildet ist, ist eine passive magnetische Lagerung mit Stabilisierung gegen Verkippung über Reluktanzkräfte möglich.

Somit sind drei Freiheitsgrade des Rotors aktiv regelbar. Bezüglich dreier weiterer Freiheitsgrade, nämlich seiner axialen Auslenkung in Richtung der Drehachse und Verkippungen bezüglich der zur Drehachse senkrechten Ebene (zwei Freiheitsgrade) ist der Rotor passiv magnetisch, das heisst nicht aktiv ansteuerbar, sondern durch Reluktanzkräfte magnetisch stabilisiert.

Damit der auftretende Axialschub, der beispielweise durch die zwischen dem Einlass und dem Auslass einer Pumpe herrschenden Druckdifferenz verursacht wird, oft nicht vollständig von den Axiallagern aufgenommen werden muss, sind in Zentrifugalpumpen ganz verschiedene Massnahmen bekannt, um den Rotor bezüglich der axialen Richtung auszubalancieren.

Besonders gravierend kann das Problem des Axialschubausgleichs somit bei Pumpen mit magnetisch gelagertem Rotor werden, insbesondere wenn die axiale Lagerung vollkommen ohne mechanische Lager magnetisch über Reluktanzkräfte erfolgt. Zur Ausbalancierung des Rotors eines solchen lagerlosen Motors stehen neben der magnetischen Reluktanzkraft nur konstruktive Massnahmen zur Verfügung, welche die Axialposition über fluiddynamische Ausgleichskräfte beeinflussen.

Die damit zusammenhängenden Probleme wurden gemäss PCT/EP2010/062 789 durch die Anmelderin inzwischen weitgehend gelöst, so dass die dort beschriebenen Massnahmen auch für die Konstruktion von Vorrichtungen unter Verwendung einer Rotationsmaschine der vorliegenden Erfindung sehr vorteilhaft angewendet werden können.

Abgesehen davon, sind die bekannten lagerlosen Motoren in verschiedenen Ausführungsformen bekannt.

So offenbart die WO-A-96/31934 z.B. eine Pumpe mit einem sogenannten Tempelmotor, bei dem die Spulenkerne des Stators jeweils die Form eines "L" haben, wobei der eine Schenkel jeweils parallel zur Rotationsachse verläuft, während der andere Schenkel radial einwärts zur Rotationsachse gerichtet ist. Der Stator, der als Lager- und Antriebsstator ausgestaltet ist, hat bevorzugt zwei Wicklungen, nämlich die Antriebswicklung und die Steuerwicklung, welche als diskrete Spulen ausgeführt sind und um die langen Schenkel der L-förmigen Spulenkerne gewickelt sind. Ein solcher Tempelmotor kommt daher ohne Wickelköpfe aus, sodass z.B. der Auslass eines Pumpengehäuses ohne räumliche Behinderung auf der Höhe des Flügelrads in Form eines radialen Auslasskanals angeordnet werden kann.

Diese Ausgestaltung als Tempelmotor, z.B. gemäss Fig. 12 der WO 96/31934, unterliegt jedoch unter anderem der Einschränkung, dass sie wegen der hohen Bauform einen relativ grossen Platzbedarf hat und im Aufbau aufwendig ist.

Als Alternative zum Tempelmotor ist daher in der EP 0 900 572 A1 der Anmelderin eine andere Rotationspumpe mit einem lagerlosen Motor gezeigt, die auf den baulich relativ grossen und aufwändigen Tempelmotor verzichtet.

Inzwischen sind eine Reihe von Ausführungsbeispielen mit unterschiedlichen Anordnungen und Ausgestaltungen der Statorzähne bekannt, z.B. auch solche, bei welchen die Statorzähne direkt die elektrischen Wicklungen tragen und im wesentlichen vollständig in der Rotorebene liegen, was besonders kompakte Bauformen ermöglicht.

Dabei ist es häufig so, dass insbesondere der Rotor ein Austauschteil ist, das je nach Anwendung mehr oder weniger häufig ersetzt werden muss. Z.B., weil der Rotor zum Pumpen von mechanisch oder chemisch sehr aggressiven Flüssigkeiten oder in sehr aggressiven Umgebungen, z.B. unter erhöhter Temperatur betrieben wird, so dass der Rotor relativ schnell verschleisst und daher häufig ersetzt werden muss.

Oder zum Beispiel bei der Behandlung empfindlicher oder hochreiner Flüssigkeiten, wie zum Beispiel bei der Verwendung in Blutpumpen oder in Bioreaktoren, oder zur Handhabung hochempfindlicher bzw. hochreiner Flüssigkeiten in der pharmazeutischen und chemischen Industrie, sowie allgemein in der Medizin bzw. der medizinischen Technik kann es beispielweise aus hygienischen Gründen bzw. um Querkontaminationen zu vermeiden notwendig sein, dass der Rotor nach einmaliger Verwendung ausgetauscht werden muss, also letztlich ein Wegwerfteil ist.

Die bekannten Rotoren der lagerlosen Scheibenmotoren sind dabei in der Praxis mit einem Permanentmagneten ausgestattet, um den Rotor insbesondere gegenüber Verkippung bzw. axiale Verschiebung mit Hilfe der oben bereits beschriebenen Reluktanzkräfte zu stabilisieren. Damit wird der Rotor zu einem sehr teueren Austausch- bzw. Wegwerfteil, da Permanentmagnete verhältnismässig teuer sind und die Materialien, wie zum Beispiel seltene Erden Materialien am Markt zu immer höheren Preisen beschafft werden müssen und die Preise weiter unaufhörlich am steigen sind. Ganz abgesehen davon, dass es allein schon aus Gründen der Nachhaltigkeit nicht sinnvoll ist, sehr hochwertige und relativ seltene Materialien, die für die Herstellung von Permanentmagneten gebraucht werden, als Wegwerfprodukt zu verwenden. Und selbst wenn die bekannten Rotoren nach Gebrauch der Wiederverwertung zugeführt werden, so dass die wertvollen Materialien nicht, oder zumindest nicht ganz verloren gehen, ist immer noch die Herstellung von Rotoren mit Permanentmagneten sehr aufwändig und auch das Recycling relativ schwierig und teuer.

Ausgehend vom Stand der Technik ist es daher eine Aufgabe der Erfindung, eine Rotationsmaschine, einen Rotor für eine Rotationsmaschine, sowie eine Vorrichtung mit einer Rotationsmaschine bzw. mit einem Rotor vorzuschlagen, wobei der Rotor günstig und einfach in der Herstellung ist, gleichzeitig wertvolle Ressourcen gespart werden und der Rotor nachhaltig und ohne grossen Aufwand auch wieder recycelt werden kann.

Die diese Aufgaben lösenden Gegenstände der Erfindung sind durch den unabhängigen Anspruch 1 gekennzeichnet. Die abhängigen Ansprüche beziehen sich auf besonders vorteilhafte Ausführungsformen der Erfindung.

Erfindungsgemäss wird somit eine Rotationsmaschine, ausgestaltet als lagerloser Motor vorgeschlagen, der einen als Lager- und Antriebsstator ausgestalteten Stator mit einer Statorwicklung und einen innerhalb des Stators magnetisch berührungslos gelagerten scheibenförmigen Rotor umfasst. Dabei ist eine axiale Höhe des Rotors kleiner oder gleich einem halben Durchmesser des Rotors und der Rotor ist in Bezug auf den Stator sowohl gegen eine Verschiebung entlang einer Rotationsachse des Rotors als auch gegen eine Verkippung aus einer Gleichgewichtslage passiv durch Reluktanzkräfte stabilisiert, wobei der Stator zur Erzeugung eines homopolaren magnetischen Flusses einen Permanentmagneten umfasst. Der Rotor ist dabei ein um ein Polstück des Stators drehbar angeordneter ringartiger Rotor und der Rotor umfasst ein ferromagnetisches Material und keinen Permanentmagneten.

Da die Rotoren der vorliegenden Erfindung keinen Permanentmagneten enthalten, ist der Rotor nunmehr ein sehr günstiges Austausch- bzw. Wegwerfteil, da der erfindungsgemässe Rotor keine seltenen und damit teuren Materialien, wie zum Beispiel seltene Erden mehr enthält. Darüber hinaus können die erfindungsgemässen Rotoren nach Gebrauch ohne grossen Aufwand recycelt und wiederverwertet werden, ohne dass wertvolle Materialien verloren gehen. Ausserdem ist die Herstellung erfindungsgemässer Rotoren deutlich weniger aufwändig, was zu deutlich reduzierten Kosten führt. Erfindungsgemässe Rotoren können einfach aus einem ferromagnetischen Material, wie z.B. im einfachsten Fall aus Eisen gefertigt werden, das eventuell je nach Anwendung noch mit einem geeigneten Material, z.B. mit einem Kunststoff beschichtet oder verkleidet wird.

Die Verwendung von Rotoren, die keinen Permanentmagneten enthalten, wird gemäss der vorliegenden Erfindung zum Einen dadurch möglich, dass über das Polstück, das in einer Ausnehmung im Rotor, die um die Rotationsachse des Rotors vorgesehen ist, den Stator und den Rotor selbst ein geschlossener magnetischer Rückschluss herstellbar ist. Dazu muss selbstverständlich neben dem Rotor sowohl der Stator selbst, als auch das Polstück des Stators zumindest ferromagnetisches Material, wie z.B. Eisen umfassen und / oder zumindest teilweise aus einem permanent magnetischen Material hergestellt sein. Erfindungsgemäss ist am Stator und / oder am Polstück des Stators mindestens ein Permanentmagnet derart vorgesehen, dass der Rotor von einem homopolaren magnetischen Fluss durchflutet wird, so dass der Rotor, wie an sich bekannt, bezüglich dreier Freiheitsgrade, nämlich seiner axialen Auslenkung in Richtung der Rotationsachse und Verkippungen bezüglich der zur Rotationsachse senkrechten Ebene (zwei Freiheitsgrade) passiv magnetisch durch Reluktanzkräfte stabilisiert ist.

Unter einem homopolaren Fluss ist dabei ein geschlossener magnetischer Flussverlauf zu verstehen, der seine Quelle in dem am Stator oder Polstück vorgesehenen Permanentmagneten hat. Dabei ist im Rotor jede zur Rotationsachse resultierende orthogonale Komponente des homopolaren Flusses immer nur in eine einzige radiale Richtung, nämlich in Bezug auf die Rotationsachse entweder nur radial nach aussen oder nur radial nach innen in Richtung zur Rotationsachse gerichtet.

In einem für die Praxis besonders bevorzugten Ausführungsbeispiel ist der Permanentmagnet das Polstück bzw. der Permanentmagnet ist am Polstück vorgesehen und entlang der Rotationsachse polarisiert. Dadurch wird ein entsprechender homopolarer Fluss induziert, der ausgehend von einem magnetischen Pol des Permanentmagneten über den Rotor und den Stator wieder zurück zum anderen magnetischen Pol des Permanentmagneten geschlossen ist.

Dabei kann zusätzlich oder in einem anderen Ausführungsbeispiel auch alternativ der Permanentmagnet in einem Umfangsbereich des Stators vorgesehen und entlang der Rotationsachse polarisiert sein und / oder der Permanentmagnet kann auch zusätzlich oder alternativ in einem Bodenbereich des Stators vorgesehen und in einer radialen Richtung orthogonal zur Rotationsachse polarisiert sein.

Da der Permanentmagnet, wie bereits erwähnt, im Prinzip an jeder geeigneten Stelle ausserhalb des Rotors am Stator oder am Polstück vorgesehen sein kann, kann der Permanentmagnet alternativ oder zusätzlich zu einem oder in dieser Anmeldung beschriebenen Varianten auch in einem Deckbereich des Stators vorgesehen und in einer radialen Richtung orthogonal zur Rotationsachse polarisiert sein. Oder aber ein Permanentmagnet kann alternativ oder zusätzlich in oder an einem Statorzahn des Stators vorgesehen werden.

Dabei können im Prinzip alle an sich bekannten, im Sinne dieser Anmeldung als lagerlose Motoren zu bezeichnenden Rotationsmaschine, die ein Polstück und einen scheibenförmigen Rotor haben, im Prinzip mit einem erfindungsgemässen Rotor ausgestattet werden, wenn nur ein Permanentmagnet geeignet ausserhalb des Rotors am Stator oder Polstück vorgesehen wird.

So kann eine erfindungsgemässe Rotationsmaschine zum Beispiel einen L-förmig ausgebildeten Statorzahn haben, wobei sich ein Schenkel des Statorzahns parallel zur Rotationsachse und sich ein anderer Schenkel des Statorzahns radial zur Rotationsachse zum Rotor hin erstreckt. Im speziellen kann eine erfindungsgemässe Rotationsmaschine zum Beispiel in Form eines Tempelmotors ausgebildet sein.

Bei einem anderen bevorzugten Ausführungsbeispiel kann der Statorzahn einer erfindungsgemässen Rotationsmaschine vollständig in einer Ebene des Rotors ausgebildet sein, so dass die Rotationsmaschine in Bezug auf die axiale Richtung eine sehr kompakte und platzsparende Bauform hat.

Damit eine magnetische Antriebskraft in Umfangrichtung auf den Rotor ausgeübt werden kann, darf die Verteilung des ferromagnetischen Materials im Rotor natürlich nicht vollkommen gleichmässig sein, sondern muss in geeigneter Weise strukturiert werden. Daher kann der Rotor zum Beispiel eine unregelmässige Aussenkontur mit einem radial nach aussen gerichteten Rotorzahn haben.

Oder aber der Rotor kann eine kreisförmige Aussenkontur haben, wobei das ferromagnetische Material des Rotors nach einem vorgebbaren Schema in Bezug auf eine Umfangsrichtung oder in Bezug auf eine radiale Richtung des Rotors geeignet verteilt am oder im Rotor vorgesehen ist. Dieses Prinzip ist grundsätzlich dem Fachmann unter dem Begriff "Synchron Reluktanzmotor" bestens bekannt.

In der Praxis ist am Stator, im Speziellen am Polstück eine Sensoreinrichtung mit einem Sensor zur Bestimmung einer Magnetfeldstärke vorgesehen, so dass im Betriebszustand eine Regelung der Position des Rotors orthogonal zur Rotationsachse möglich ist.

Die aktive Regelung der Position in jede der beiden Radialrichtungen des Rotors erfolgt dabei bevorzugt mittels mindestens eines am Statorumfang oder am Polstück angebrachten Sensors pro Richtung, der die Distanz des Rotors zum Stator misst. Alternativ ist es möglich, aus Gründen verbesserter Linearität oder Auflösung auch zwei oder mehr Sensoren pro Richtung zu platzieren, wobei je nach Platzierung in dem Fachmann bekannter Weise durch Differenz- und / oder Summenbildung die Position in ebendiese Richtung ermittelt wird.

Eine Bewegung des Rotors von der Sollposition des Rotors in eine bestimmte Richtung, z.B. durch eine Störeinwirkung, führt dann zum Beispiel zu einer Verkleinerung des Signals am Distanzsensor, was dann zu einer Abschwächung des Feldes im Luftspalt auf eben dieser Seite und das wiederum zu einer Verstärkung des Feldes auf der gegenüberliegenden Seite durch entsprechend geregelte stromdurchflossene und felderzeugende Spulen führt. Dies bewirkt eine Bewegung des Rotors in Richtung zur Sollposition und somit eine aktive radiale Stabilisierung. Diese Art der Regelung ist für lagerlose Motoren an sich bekannt und braucht nicht weiter im Detail erläutert zu werden.

Der Sensor ist dabei bevorzugt ein Magnetfeldsensor, insbesondere ein Hallsensor oder ein magnetoresistiver Sensor, oder kann auch ein Wirbelstromsensor sein, und / oder die Sensoreinrichtung kann auch durch ein Array einer Mehrzahl von Sensoren gebildet werden.

Die Erfindung betrifft weiter einen scheibenförmigen Rotor für eine Rotationsmaschine gemäss der vorliegenden Erfindung, wobei im Rahmen dieser Anmeldung mit dem Begriff "scheibenförmiger Rotor" ein Rotor gemeint ist, dessen axiale Höhe kleiner oder gleich seinem halben Durchmesser ist. Dadurch ist gewährleistet, dass der Rotor gleichzeitig axial und gegen Verkippung allein durch magnetische Reluktanzkräfte, die in der Statorebene zwischen Stator Rotor wirken, und damit rein passiv stabilisierbar ist. Ein erfindungsgemässer Rotor ist dabei ein ringartiger Rotor mit einer um eine Rotationsachse des Rotors vorgesehenen Ausnehmung, in welcher im Einbauszustand das Polstück aufnehmbar ist. Erfindungsgemäss umfasst der Rotor ein ferromagnetisches Material und keinen Permanentmagneten.

Darüber hinaus betrifft die Erfindung eine Vorrichtung, insbesondere Wafer Bearbeitungsanlage, Bioreaktoranlage, Pumpe, Mischer oder eine andere Vorrichtung mit einer Rotationsmaschine und / oder einem Rotor der Erfindung.

Im Folgenden wird die Erfindung anhand von Ausführungsbeispielen unter Zuhilfenahme der Zeichnung näher erläutert. Die schematische Zeichnung zeigt teilweise im Schnitt:
- Fig. 1a:: ein erstes Ausführungsbeispiel einer erfindungsgemässen Rotationsmaschine;
- Fig. 1b:: einen senkrechten Schnitt entlang der Schnittlinie I-I gemäss Fig. 1a;
- Fig. 1c:: einen waagrechten Schnitt entlang der Schnittlinie I-I gemäss Fig. 1 a zur Demonstration der Positionsregelung des Rotors in X-Richtung;
- Fig. 1d:: einen waagrechten Schnitt entlang der Schnittlinie I-I gemäss Fig. 1 a zur Demonstration der Positionsregelung des Rotors in Y-Richtung;
- Fig. 2a:: ein zweites Ausführungsbeispiel einer erfindungsgemässen Rotationsmaschine;
- Fig. 2b:: einen waagrechten Schnitt entlang der Schnittlinie II-II gemäss Fig. 2a zur Demonstration der Positionsregelung des Rotors in X-Richtung;
- Fig. 2c:: eine Demonstration der Positionsregelung gemäss Fig. 2a in Y-Richtung;
- Fig. 3:: ein drittes Ausführungsbeispiel einer erfindungsgemässen Rotationsmaschine mit Permanentmagnet im Umfangsbereich;
- Fig. 4:: ein viertes Ausführungsbeispiel einer erfindungsgemässen Rotationsmaschine mit Permanentmagnet im Deckbereich;
- Fig. 5a:: ein erstes Ausführungsbeispiel eines Rotors mit kreisförmiger Aussenkontur;
- Fig. 5b:: ein anderes Ausführungsbeispiel gemäss Fig. 5a;
- Fig. 5c:: ein zweites Ausführungsbeispiel eines Rotors mit kreisförmiger Aussenkontur;
- Fig. 5d:: ein drittes Ausführungsbeispiel eines Rotors mit kreisförmiger Aussenkontur;
- Fig. 6a:: ein Schnitt durch eine erfindungsgemässe Pumpe;
- Fig. 6b:: einen waagrechten Schnitt entlang der Schnittlinie III-III gemäss Fig. 6a.

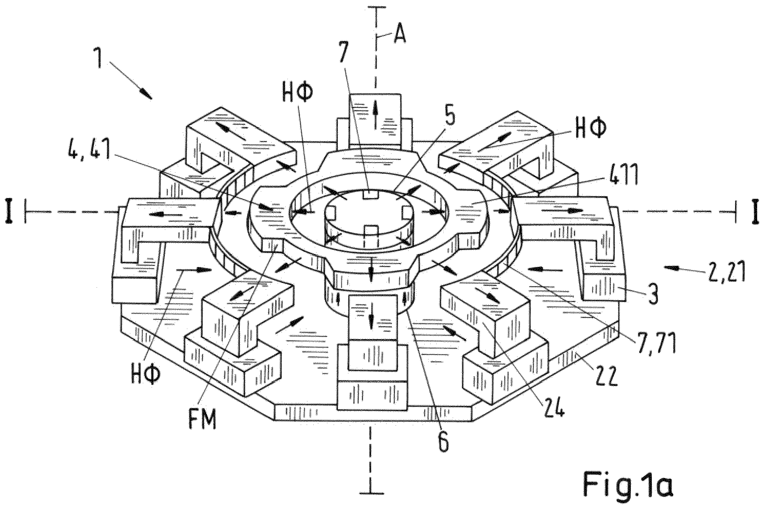

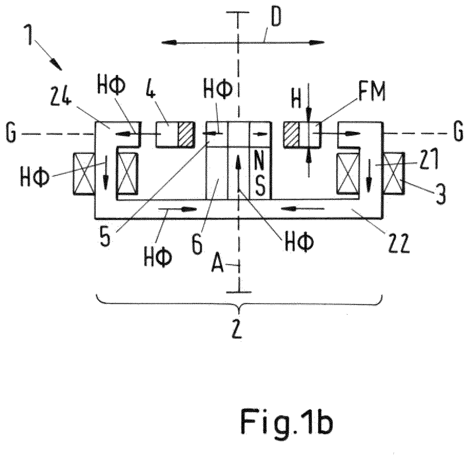

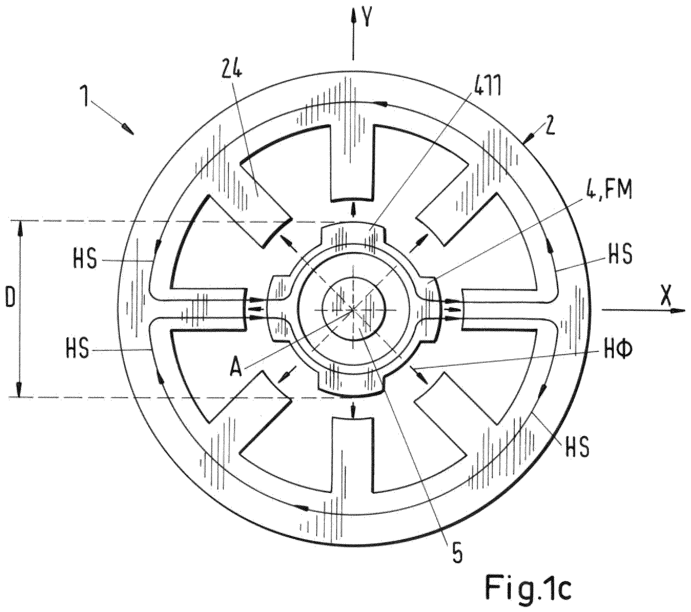

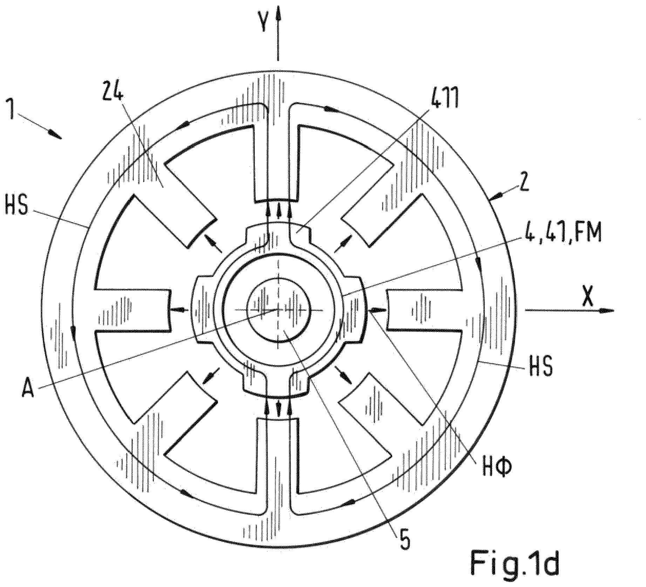

Anhand der schematischen Fig. 1a bis Fig. 1d ist ein erstes Ausführungsbeispiel einer erfindungsgemässen Rotationsmaschine dargestellt, die im Folgenden gesamthaft mit dem Bezugszeichen 1 bezeichnet ist. Dabei zeigt die Fig. 1b einen senkrechten Schnitt entlang der Schnittlinie I-I gemäss Fig. 1a, und die Fig. 1c und Fig. 1d zeigen jeweils einen waagrechten Schnitt entlang der Schnittlinie I-I gemäss Fig. 1a zur Demonstration der Positionsregelung des Rotors in X-Richtung bzw. in Y-Richtung in der X-Y-Ebene senkrecht zur Rotationsachse des Rotors.

Die erfindungsgemässe Rotationsmaschine 1 gemäss Fig. 1 ist in an sich bekannter Weise, wie eingangs bereits beschrieben, als lagerloser Motor, umfassend einen als Lager- und Antriebsstator ausgestalteten Stator 2 mit einer Statorwicklung 3 und einem innerhalb des Stators 2 magnetisch berührungslos gelagerten scheibenförmigen Rotor 4 ausgestaltet. Wie ebenfalls bereits erwähnt, ist ein scheibenförmiger Rotor im Rahmen dieser Anmeldung ein Rotor, dessen axiale Höhe H kleiner oder gleich einem halben Durchmesser D des Rotors 4 ist.

Dabei ist der Durchmesser D des Rotors 4 definiert als der am Rotor 4 maximal vorkommende Abstand zwischen zwei in Bezug auf eine Rotationsachse A des Rotors 4 radial aussen liegenden Begrenzungen eines am oder im Rotor 4 vorgesehen ferromagnetischen Materials. Dies wird im Folgenden anhand der Zeichnungen noch näher erläutert werden.

Der Rotor 4 ist dabei, wie bei lagerlosen Motoren im Sinne der Anmeldung üblich, in Bezug auf den Stator 2 sowohl gegen eine Verschiebung entlang einer Rotationsachse A als auch gegen eine Verkippung gegen die Rotationsachse A aus einer Gleichgewichtslage G passiv durch Reluktanzkräfte stabilisiert, und der Stator 2 umfasst zur Erzeugung eines homopolaren magnetischen Flusses HΦ einen Permanentmagneten 6. Erfindungsgemäss ist der Rotor 4 ein um ein Polstück 5 des Stators 2 drehbar angeordneter ringartiger Rotor 4 und der Rotor 4 umfasst ein ferromagnetisches Material FM und keinen Permanentmagneten.

Wie bereits ausführlich erläutert, ist es ein wesentliches Charakteristikum des lagerlosen Motors, dass er keine separaten Magnetlager für den Rotor 4 aufweist. Dazu umfasst der Stator 2 in geeigneter Anordnung einen Permanentmagneten 6 und der Rotor 4 umfasst zur Realisierung einer passiven magnetischen Lagerung ein ferromagnetisches Material FM aber keinen Permanentmagneten.

Dabei ist für die erfindungsgemässe Rotationsmaschine 1 das Polstück 5 wesentlich. Die Verwendung von Rotoren 4, die keinen Permanentmagneten enthalten, wird gemäss der vorliegenden Erfindung nämlich dadurch möglich, dass über das Polstück 5, das in einer Ausnehmung im Rotor 4, die um die Rotationsachse A des Rotors vorgesehen ist, den Stator 2 und den Rotor 4 selbst ein geschlossener magnetischer Rückschluss herstellbar ist. Daher ist es auch zwingend erforderlich, dass der Rotor 4 als ringartiger Rotor 4 mit der erwähnten Ausnehmung für das Polstück 5 ausgestaltet ist.

Dazu muss selbstverständlich neben dem Rotor 4 sowohl der Stator 2 als auch das Polstück 5 des Stators 2 zumindest teilweise ein ferromagnetisches Material FM, wie z.B. Eisen umfassen und / oder zumindest teilweise aus einem permanent magnetischen Material hergestellt sein. Am Stator 2 und / oder am Polstück 5 des Stators 2 ist daher mindestens ein permanentmagnetisches Material FM, also ein Permanentmagnet derart vorgesehen, dass der Rotor 4 vom homopolaren magnetischen Fluss HΦ durchflutet wird, so dass der Rotor 4, wie an sich bekannt, bezüglich dreier Freiheitsgrade, nämlich einer axialen Auslenkung in Richtung der Rotationsachse A und einer Verkippungen bezüglich der zur Rotationsachse A senkrechten X-Y-Ebene (zwei Freiheitsgrade) passiv magnetisch durch Reluktanzkräfte stabilisiert ist.

Wie bereits eingangs erläutert, ist dabei unter einem homopolaren Fluss HΦ ein geschlossener permanent magnetischer Flussverlauf zu verstehen, der seine Quelle in dem am Stator 2, im Speziellen am Polstück 5 vorgesehenen Permanentmagneten hat. Dabei ist im Rotor 4 jede zur Rotationsachse A resultierende orthogonale Komponente des homopolaren Flusses HΦ bei ein und demselben Motor immer nur in eine einzige radiale Richtung, nämlich in Bezug auf die Rotationsachse A entweder nur radial nach aussen oder nur radial nach innen in Richtung zur Rotationsachse gerichtet. Es versteht sich, dass der homopolare Fluss natürlich im Rotor 4 einer bestimmten erfindungsgemässen Rotationsmaschine 1 von der Rotationsachse A aus radial nach aussen gerichtet sein kann, und bei einem anderen Ausführungsbeispiel radial nach innen gerichtet sein kann, was letztlich von der magnetischen Orientierung, also der Polarisationsrichtung des am Stator 2 oder Polstück 5 vorgesehen Permanentmagneten abhängt.

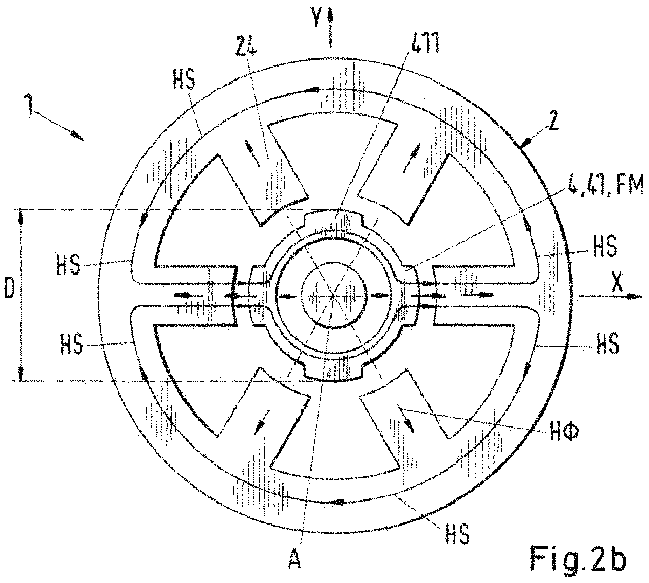

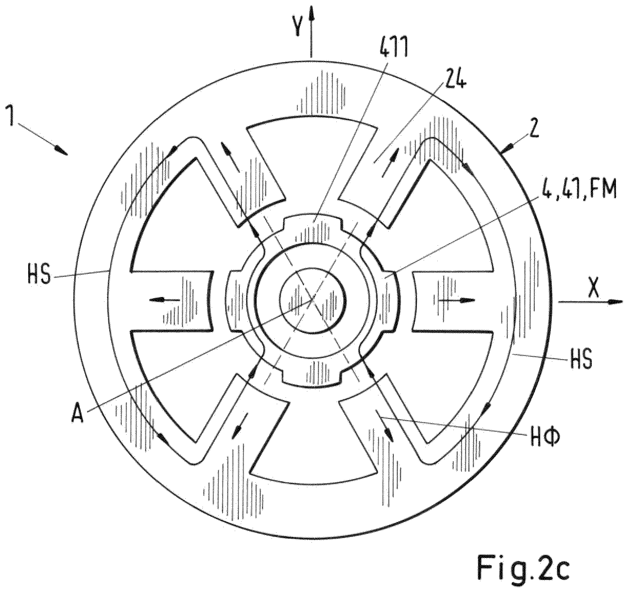

Es sei an dieser Stelle deutlich darauf hingewiesen, dass der durch den Permanentmagneten erzeugte homopolare Fluss HΦ streng zu unterscheiden ist vom Steuerfluss HS, der durch die Statorwicklungen 3 erzeugt wird, dem homopolaren Fluss HΦ überlagert ist und zur Steuerung und / oder Regelung der radialen Position des Rotors 4 in der X-Y-Ebene, die senkrecht zur Rotationsachse A orientiert ist, dient. Die Wirkung des Steuerflusses HS wird später bei der Diskussion der Fig. 1c und Fig. 1 d bzw. der Fig. 2b und Fig. 2c noch im Detail erläutert werden.

Bei dem speziellen Ausführungsbeispiel dargestellt in den Fig. 1a - Fig. 1d ist der Permanentmagnet 6 am Polstück 5, darstellungsgemäss unter dem Polstück 5 vorgesehen und entlang der Rotationsachse A polarisiert.

Die der Statorzähne 24 sind am Stator 2 derart L-förmig ausgebildet, dass sich ein Schenkel des Statorzahns 24 parallel zur Rotationsachse A und sich ein anderer Schenkel desselben Statorzahns 24 radial zur Rotationsachse A zum Rotor 4 hin erstreckt.

Der Rotor 4 hat dabei eine unregelmässige Aussenkontur 41 mit einem radial nach aussen gerichteten Rotorzahn 411, wobei das ferromagnetische Material FM zumindest in den Rotorzähnen 411 vorgesehen ist, so dass das ferromagnetische Material FM des Rotors 4 dadurch nach einem vorgebbaren Schema zumindest in Bezug auf eine Umfangsrichtung U am oder im Rotor 4 verteilt ist, so dass mittels eines durch die Statorwicklung 3 erzeugten magnetischen Antriebsfeldes der Rotor 4 in Rotation um die Rotationsachse A versetzt werden kann.

Zur Steuerung und / oder Regelung der radialen Position des Rotors 4 in der X-Y-Ebene, die im Schnittbild der Fig. 2c und Fig. 2d zu sehen ist, und die senkrecht zur Rotationsachse A orientiert ist, ist am Stator 2, hier im Speziellen auch am Polstück 5 eine Sensoreinrichtung 7 mit einem Sensor zur Bestimmung einer Magnetfeldstärke vorgesehen, wobei in einem anderen Ausführungsbeispiel selbstverständlich auch nur z.B. nur am Polstück 5 oder nur am oder nur zwischen zwei Statorzähnen 24 eine Sensoreinrichtung 7 vorgesehen sein kann. Der Sensor ist dabei bevorzugt ein Magnetfeldsensor, insbesondere ein Hallsensor, oder ein magnetoresistiver Sensor, oder ein Wirbelstromsensor ist, wobei die Sensoreinrichtung 7 zum Beispiel auch wie in Fig. 1 a schematisch dargestellt durch ein Array 71 von Sensoren gebildet sein kann.

Der Stator 2 umfasst dabei wie erwähnt die elektrische Statorwicklung 3, mit welcher ein elektromagnetisches Drehfeld erzeugbar ist, das zum einen ein Drehmoment auf den Rotor 4 ausübt, das im Betriebszustand dessen Rotation um die Rotationsachse A antreibt, und welches zum anderen eine beliebig einstellbare Querkraft auf den Rotor 4 ausübt, sodass dessen radiale Position bezüglich der zur Rotationsachse A senkrechten X-Y-Ebene vorgebbar bzw. aktiv steuerbar oder regelbar ist. Somit ist der Rotor 4 im Betriebszustand mittels der elektrischen Statorwicklungen 3 des Stators 2 bezüglich dreier Freiheitsgrade, nämlich der Rotation um die Drehachse sowie der radialen Position in der zur Drehachse senkrechten Ebene (zwei Freiheitsgrade) aktiv ansteuerbar bzw. antreibbar.

Im speziellen Ausführungsbeispiel einer erfindungsgemässen Rotationsmaschine 1 gemäss Fig. 1a bis Fig. 1d ist am Polstück 5 eine Sensoreinrichtung 7 in Form von vier Magnetfeldsensoren, z.B. Hall Sensoren oder magnetoresistive Sensoren, die das unipolare Magnetfeld messen, deutlich zu erkennen. Wenn der Rotor 4 beispielsweise darstellungsgemäss nach rechts ausgelenkt wird, vergrössert sich der Luftspalt auf der rechten Seite zwischen dem Polstück 5 und der Innenwand der Ausnehmung des ferromagnetischen Ringrotors 4 und der Luftspalt auf der darstellungsgemäss linken Seite zwischen dem Polstück 5 und der Innenwand des ferromagnetischen Rotorrings 4 verringert sich. Dadurch wird das Magnetfeld auf der rechten Seite kleiner und das Magnetfeld auf der linken Seite grösser. Die Magnetfelder werden durch den darstellungsgemäss rechten und linken Magnetfeldsensor gemessen und die Differenz des Signals des linken Sensors und des rechten Sensors bildet ein Signal für die X-Position (nach rechts zeigend) des Rotors. In gleicher Weise kann auch die Y-Position aus dem vorderen und hinteren Sensorsignal bestimmt werden.

Die zugehörigen magnetischen Flussverläufe der Steuerflüsse HS sind exemplarisch für die Steuerung bzw. Regelung der Position des Rotors 4 in Fig. 1c für die X-Richtung und in Fig. 1d für die Y-Richtung schematisch dargestellt. Dabei ist aus Gründen der Übersichtlichkeit jeweils nur ein Paar von Flusslinien HS dargestellt, die jeweils in der X-Y-Ebene in sich geschlossene Flusslinien des Steuerflusses HS bilden, und die dem homopolaren magnetischen Fluss HΦ überlagert sind, wobei der homopolaren magnetische Fluss HΦ, wie besonders gut im senkrechten Schnitt der Fig. 1b zu sehen ist, senkrecht zur X-Y-Ebene über das Polstück 5 ebenfalls in sich geschlossen ist. Der Fachmann versteht dabei, dass ebenso gut selbstverständlich auch andere Paare von Flusslinien des Steuerflusses HS in den Fig. 1c und Fig. 1g hätten dargestellt werden können und uns solche weiteren Paare von Flusslinien des Steuerflusses HS im Betriebszustand der Rotationsmaschine 1 selbstverständlich auch vorhanden sind.

Es versteht sich von selbst, dass anstelle der Magnetfeldsensoren gemäss Fig. 1a bzw. Fig. 1b auch Wirbelstromsensoren vorteilhaft verwendet werden können, wobei dies beispielsweise als Leiterbahnen einer starren oder flexiblen Leiterplatte ausgeführt sein können.

Im speziellen Ausführungsbeispiel gem. Fig. 1a sind zusätzlich zu den Sensoren am Polstück 5 in vier der Zwischenräume zwischen benachbarten Statorzähnen 24 Anordnungen von mehreren Magnetfeldsensoren, die hier je ein Magnetfeldsensorarray bilden, vorgesehen. Ein solches Magnetfeldsensorarray kann aus mehreren Einzelsensoren bestehen oder es können mehrere Einzelsensoren z.B. auf einem Chip integriert sein.

Je nachdem ob ein Magnetfeldsensor (im Magnetfeldsensorarray) einem Rotorzahn 411 (der dargestellte Rotor 4 hat vier Rotorzähne) oder einer Rotornut (der dargestellte Rotor 4 hat ebenfalls vier Rotornuten) gegenüberliegt, ist das gemessene Magnetfeld grösser oder kleiner. Aus den Signalen der einzelnen Magnetfeldsensoren kann damit die Winkellage des Rotors festgestellt werden. Um Schwankungen, welche von der radialen Auslenkung des Rotors herrühren zu kompensieren, kann bevorzugt jeweils die Summe von 2 X 180° gegenüberliegenden Sensoren gebildet werden. Die Summe ist dann von Schwankungen der Radialposition weitgehend unabhängig und hängt praktisch nur noch von der Winkellage des Rotors ab. Natürlich kann auch die radiale Position, welche mit den innen angeordneten Magnetfeldsensoren bestimmt wurde, zur Kompensation der Radialposition des Rotors 4 herangezogen werden. Dadurch wird der Aufwand für die Hardware geringer, der Aufwand auf Softwareseite jedoch etwas grösser. Falls das Magnetfeldsignal zu klein ist, können zusätzlich radial magnetisierte Magnetsegmente (in dieselbe Richtung wie das unipolare Magnetfeld magnetisiert) hinter den Magnetfeldsensorarrays angeordnet werden. Es ist auch möglich kleine Einzelmagnete hinter den Sensoren anzuordnen. Anstelle der Magnetfeldsensoren kann natürlich auch ein Array von Wirbelstromsensoren angeordnet werden, die beispielsweise als Leiterbahnen einer starren oder flexiblen Leiterplatte ausgeführt sein können.

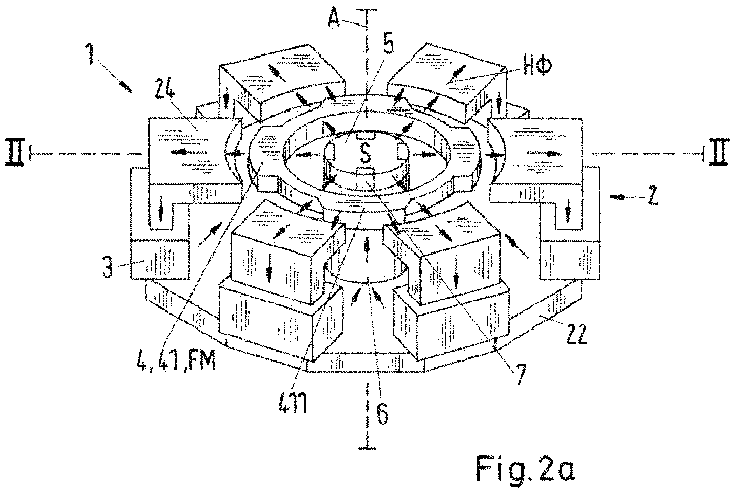

Die Fig. 2a bis Fig. 2c zeigen ein zweites Ausführungsbeispiel einer erfindungsgemässen Rotationsmaschine 1, wobei die Fig. 2b und Fig. 2c jeweils einen waagrechten Schnitt entlang der Schnittlinie II-II gemäss Fig. 2a zur Demonstration der Positionsregelung des Rotors zeigen. Im Fall der Fig. 2b die Positionsregelung in X-Richtung in der X-Y-Ebene senkrecht zur Rotationsachse des Rotors und im Fall der Fig. 2c die Positionsregelung gemäss Fig. 2a anhand zweier anderer Polpaare.

Die Fig. 2a zeigt ein weiteres Ausführungsbeispiel einer erfindungsgemässen Rotationsmaschine 1, die sich von demjenigen gemäss Fig. 1a nur dadurch unterscheidet, dass hier eine geringere Anzahl von Statorzähnen 24 vorgesehen ist als bei Beispiel der Fig. 1a. Dabei sind die Statorzähne gemäss Fig. 2a derart angeordnet, dass es keine Rotorstellung gibt, in der jedem Rotorzahn 411 ein Statorzahn 24 gegenübersteht, anders als bei dem Beispiel der Fig. 1a, bei welcher es aufgrund der Anordnung der Statorzähne 24 am Stator 2 Einstellungen des Rotors 4 gibt, bei welcher jedem Rotorzahn 411 ein Statorzahn 24 gegenübers steht.

Das hat natürlich Auswirkungen auf den Verlauf der der magnetischen Flussverläufe der Steuerflüsse HS, welche für die Rotationsmaschine der Fig. 2a exemplarisch in Fig. 2b und Fig. 2d dargestellt sind. Dabei ist auch hier aus Gründen der Übersichtlichkeit jeweils nur ein Paar von Flusslinien HS dargestellt, die jeweils in der X-Y-Ebene in sich geschlossene Flusslinien des Steuerflusses HS bilden, und die dem homopolaren magnetischen Fluss HΦ überlagert sind. Auch bei diesem Beispiel versteht der Fachmann sofort, dass ebenso gut selbstverständlich auch andere Paare von Flusslinien des Steuerflusses HS in den Fig. 2b und Fig. 2c hätten dargestellt werden können und weitere Paare von Flusslinien des Steuerflusses HS im Betriebszustand der Rotationsmaschine 1 selbstverständlich auch vorhanden sind.

Im Übrigen ist klar, dass die Erfindung nicht auf Rotationsmaschinen 1 mit einer bestimmten Anzahl von Statorzähnen 24 beschränkt ist, und eine beliebige gerade und ungerade Zahl von Statorzähnen 24 möglich ist, wobei in der Praxis bevorzugt mindestens drei Statorzähne 24 vorhanden sind. Selbstverständlich ist auch die Form des Rotors 4 keinesfalls auf die in der Anmeldung exemplarisch gezeigten Ausführungsformen beschränkt und kann im Prinzip beliebig ausgebildet sein, wenn sich nur ein Drehmoment auf den Rotor 4 ausüben lässt und er über Reluktanzkräfte passiv magnetisch stabilisierbar ist.

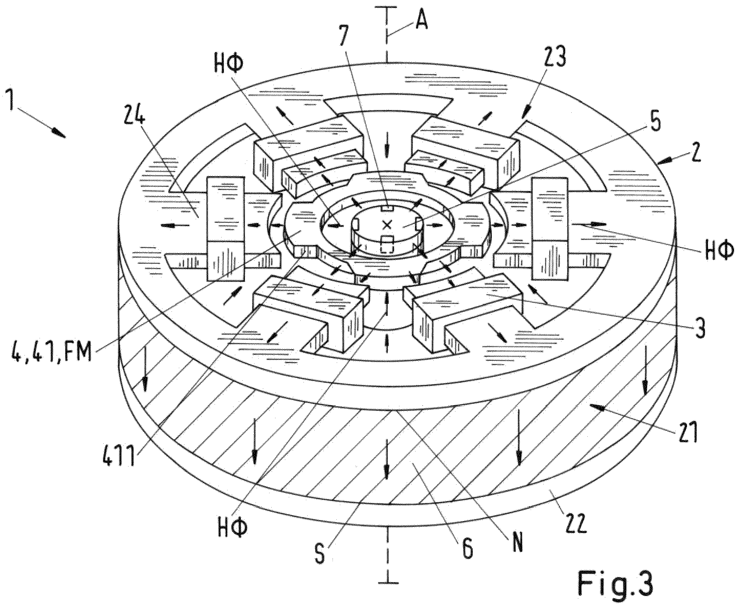

Anhand der Fig. 3 wird exemplarisch ein drittes Ausführungsbeispiel einer erfindungsgemässen Rotationsmaschine 1 erläutert, bei welcher der Permanentmagnet 6 nicht am Polstück 5, sondern in einem Umfangsbereich 21 des Stators 2 in Form eines Ringmagnets 6 vorgesehen ist. Das heisst, zwischen einem darstellungsgemäss oberen Deckbereich 23 und einem Bodenbereich 22 des Stators 2 ist hier ein ringförmiger Permanentmagnet 6 vorgesehen, der in Richtung der Rotationsachse A polarisiert wird und so den homopolaren magnetischen Fluss HΦ über das Polstück 5 und den Rotor 4 induziert.

Der Fachmann versteht, dass beim Ausführungsbeispiel der Fig. 3 anstatt des Ringmagnets 6 zum Beispiel auch eine Mehrzahl von einzelnen Permanentmagneten 6 über den Umfang des Stators 2 verteilt vorgesehen sein können, die in Richtung der Rotationsachse A polarisiert sind.

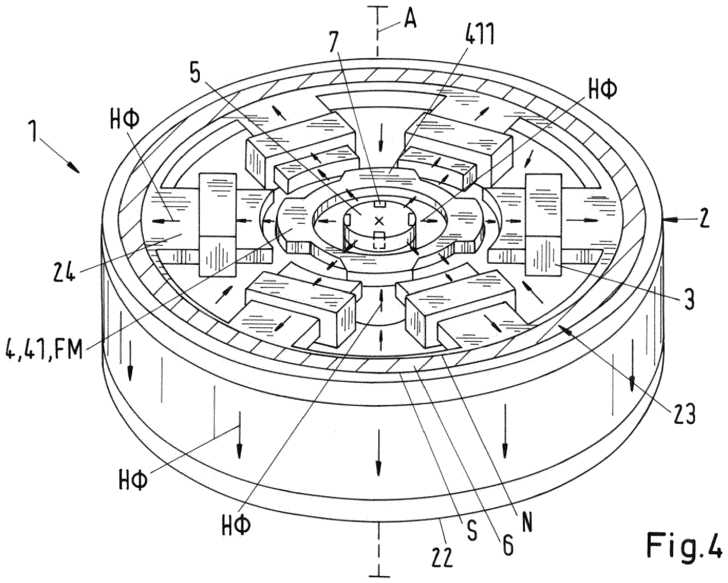

Die Fig. 4 zeigt ein viertes Ausführungsbeispiel einer erfindungsgemässen Rotationsmaschine 1, wobei der Permanentmagnet 6 in Form eines in radialer Richtung magnetisch polarisierten Magnetrings 6 im Deckbereich 23 des Stators 2 ausgestaltet ist, wobei in einem anderen Ausführungsbeispiel, in völlig analoger Weise der in radialer Richtung magnetisch polarisierte Magnetrings 6 auch alternativ oder zusätzlich im Bodenbereich 22 des Stators 2 ausgebildet sein könnte.

Auch bei diesen Ausführungsformen ist es selbstverständlich möglich, dass anstatt einem radial magnetisch polarisierten Magnetring 6 im Deckbereich 23 und / oder im Bodenbereich 22 des Staor 2, im Deckbereich 23 und / oder im Bodenbereich 22 über die Umfangsrichtung U verteilt einzelne Magnete 6 vorgesehen sind, die senkrecht zur Rotaionsachse A in radialer Richtung polarisiert sind.

Der besondere Vorteil der Ausführungsbeispiele der Fig. 3 und Fig. 4 liegt dabei unter anderem darin, dass die Statorzähne 24 vollständig in der X-Y-Ebene des Rotors angeordnet sind, also nicht L-förmig sind, was eine besonders kompakte Bauhöhe in Bezug auf die Richtung der Rotationsachse A erlaubt. Dabei ist es im Prinzip natürlich möglich, dass auch die Ausführungsbeispiele, z.B. gemäss Fig. 1 a oder Fig. 2a, bei welchen der Permanentmagnet 6 am Polstück 5 ausgebildet ist, mit Statorzähnen 24 realisierbar ist, die wie bei den Fig. 3 und Fig. 4 vollständig in der X-Y-Ebene des Rotors 4 ausgebildet sind.

Auch ist klar, dass die Permanentmagneten 6 im Prinzip überall am Stator, also auch z.B. an den Statorzähnen 24 vorgesehen sein können und alle möglichen Ausführungsbeispiele auch kombiniert werden können, beispielweise um die Stärke des homopolaren Flusses HΦ auf eine vorgebbare Stärke einzustellen.

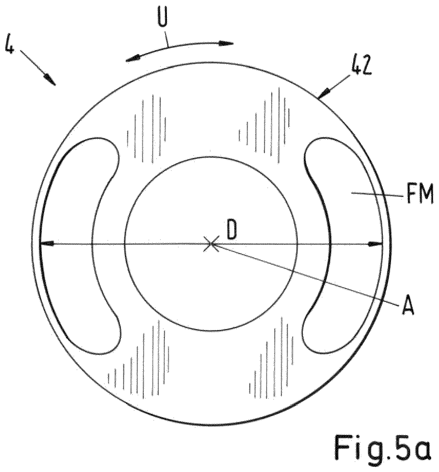

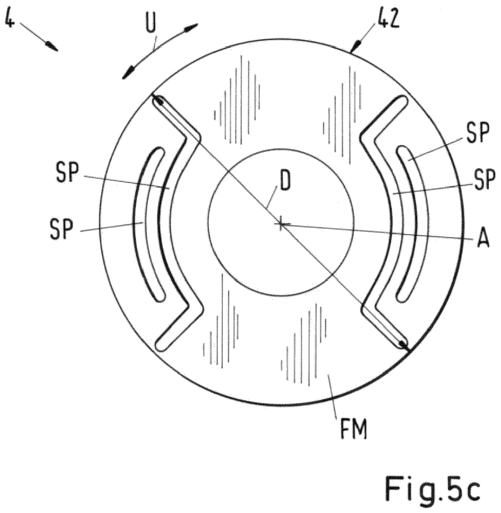

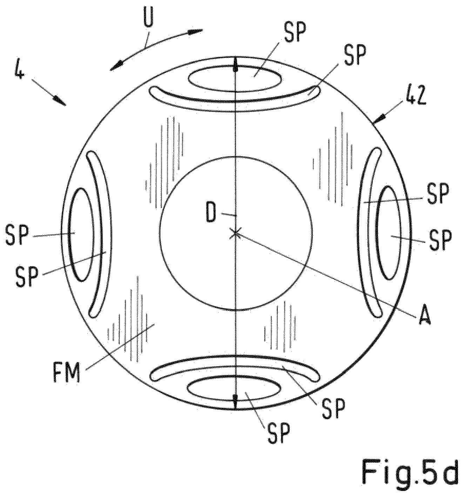

Anhand der Fig. 5a bis Fig. 5d werden exemplarisch noch einige alternative Ausführungsvarianten erfindungsgemässer Rotoren 4 mit kreisförmiger Aussenkontur 42 beschrieben.

Bei den Rotoren 4 gemäss Fig. 5a bis Fig. 5d ist der Ringrotor 4 sowohl an der inneren Ausnehmung als auch an der äusseren Kontur 42 ringförmig ausgeführt.

Beim Beispiel der Fig. 5a ist der Rotor 4 nur in den mit FM bezeichneten nierenförmigen Bereichen aus einem ferromagnetischem Material FM aufgebaut und ansonsten aus einem anderen Material, z.B. aus einem Kunststoff.

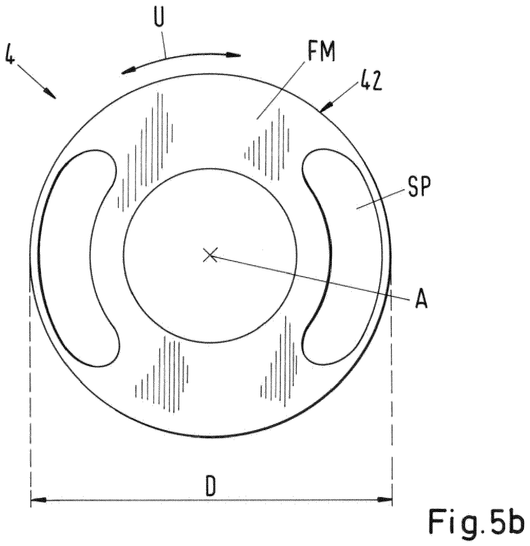

Für die meisten Fälle werden in der Praxis jedoch Ausführbeispiele gemäss Fig. 5b bis Fig. 5d ausgewählt werden, bei welchen die magnetische Anisotropie des Rotors 4 dadurch erreicht wird, dass magnetische Flusssperren SP in den Rotor 4 eingefügt sind. Die Flusssperren SP sind zum Beispiel "Ausnehmungen" in Form von Schlitzen oder Sperrflächen in der ansonsten ferromagnetischen Struktur FM des Rotors 4, welche mit Luft oder anderem nicht ferromagnetischen Material, z.B. Kunststoff gefüllt sein können. Da der magnetische Fluss durch das nicht ferromagnetische Material einen sehr hohen magnetischen Widerstand erfährt, während das ferromagnetische Material FM als "magnetischer Leiter" betrachtet werden kann, kann dem Rotor 4 durch geeignete Dimensionierung dieser Flusssperren SP eine beliebige (2p - polige) Anisotropie aufgezwungen werden. Insbesondere lässt sich mit solchen Flusssperren SP die Flussführung für die Anforderungen sowohl des Antriebs als auch der magnetischen Lagerung optimieren. So lässt sich beispielsweise ein eventuell mögliches sogenanntes Nutrasten, das, wie der Fachmann weiss, zum Beispiel dem in Figur 1 gezeigten Rotor 4 (kreuzförmig) vorkommen kann, stark reduzieren. Auch ist es prinzipiell möglich, 4-polige Rotoren 4 zu realisieren, welche sowohl vom Antrieb her als auch von der passivmagnetischen Stabilisierung der Verkippung und der Axiallage des Rotors 4, und auch von der aktiv magnetischen Stabilisierung der Radialposition des Rotors 4 her optimal sind.

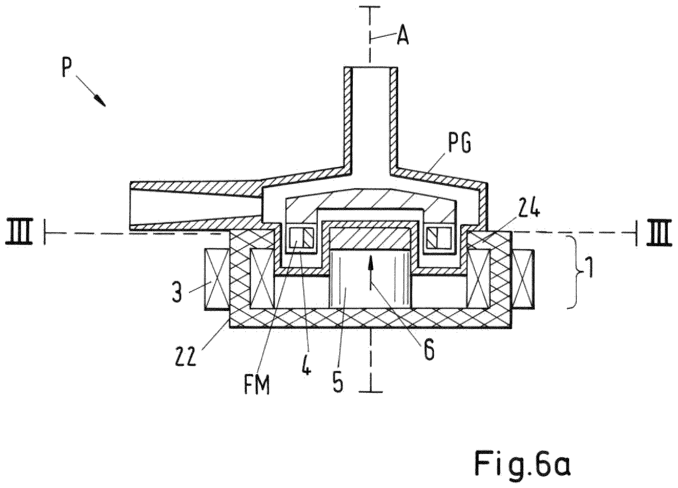

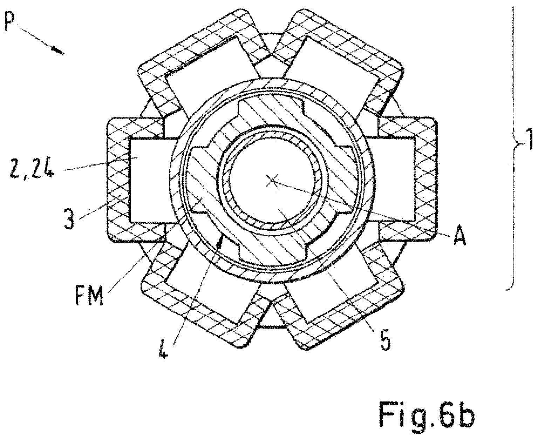

Die Fig. 6a und Fig. 6b zeigen schliesslich ein für die Praxis sehr wichtiges Anwendungsbeispiel für eine erfindungsgemässe Rotationsmaschine 1 in Form einer Pumpe P, z.B. Zentrifugalpumpe, wobei die Fig. 6b einen waagrechten Schnitt entlang der Schnittlinie III-III gemäss der Schnittzeichnung Fig. 6a der Pumpe P zeigt.

Die Pumpe P gemäss Fig. 6a bzw. Fig. 6b hat als Antrieb eine Rotationsmaschine 1 mit L-förmigen Statorzähnen 24, wie zum Beispiel anhand der Fig. 1a oder 2a erläutert. Der Permanentmagnet 6 liegt hier zentral am Polstück 5. Es versteht sich von selbst, dass natürlich auch Ausführungsvarianten mit allen anderen erfindungsgemässen Rotationsmaschinen möglich sind.

Wie gut zu erkennen ist, kann das Pumpengehäuse PG mit darin angeordnetem Rotor 4, das in an sich bekannter Weise einen Einlass und einen Auslass für ein zu pumpendes Fluid aufweist, vollständig vom Statorteil etwa auf Höhe der Schnittlinie III-III abgetrennt werden. Das Pumpengehäuse PG ist dabei, abgesehen vom Einlass und Auslass, hermetisch gegen die Umwelt, vor allem auch gegen den Stator 2, abgeschlossen. Wenn der Rotor 4 ausgetauscht werden muss, kann das Pumpengehäuse PG vom Stator 2 abgehoben und geöffnet werden und einfach ein neuer Rotor eingesetzt werden. Selbstverständlich kann auch die Pumpe insgesamt wegwerfbar, also Disposible bzw. als Einmalteil ausgestaltet sein.

## Patentansprüche

1. Rotationsmaschine, ausgestaltet als lagerloser Motor, umfassend einen als Lager- und Antriebsstator ausgestalteten Stator (2) mit einer Statorwicklung (3) und einen innerhalb des Stators (2) magnetisch berührungslos gelagerten scheibenförmigen Rotor (4), wobei eine axiale Höhe (H) des Rotors (4) kleiner oder gleich einem halben Durchmesser (D) des Rotors (4) ist, und der Rotor (4) in Bezug auf den Stator (2) sowohl gegen eine Verschiebung entlang einer Rotationsachse (A) des Rotors (4) als auch gegen eine Verkippung aus einer Gleichgewichtslage (G) passiv durch Reluktanzkräfte stabilisiert ist, und der Stator (2) zur Erzeugung eines homopolaren magnetischen Flusses (HΦ) einen Permanentmagneten (6) umfasst, **dadurch gekennzeichnet, dass** der Rotor (4) ein um ein Polstück (5) des Stators (2) drehbar angeordneter ringartiger Rotor (4) ist und der Rotor (4) ein ferromagnetisches Material (FM) und keinen Permanentmagneten umfasst.

2. Rotationsmaschine nach Anspruch 1, wobei der Permanentmagnet (6) das Polstück (5) ist oder am Polstück (5) vorgesehen ist, und entlang der Rotationsachse (A) polarisiert ist.

3. Rotationsmaschine nach Anspruch 1 oder 2, wobei der Permanentmagnet (6) in einem Umfangsbereich (21) des Stators (2) vorgesehen und entlang der Rotationsachse (A) polarisiert ist.

4. Rotationsmaschine nach einem der vorangehenden Ansprüche, wobei der Permanentmagnet (6) in einem Bodenbereich (22) des Stators (2) vorgesehen und in einer radialen Richtung orthogonal zur Rotationsachse (A) polarisiert ist.

5. Rotationsmaschine nach einem der vorangehenden Ansprüche, wobei der Permanentmagnet (6) in einem Deckenbereich (23) des Stators vorgesehen und in einer radialen Richtung orthogonal zur Rotationsachse (A) polarisiert ist.

6. Rotationsmaschine nach einem der vorangehenden Ansprüche, wobei der Permanentmagnet (6) in oder an einem Statorzahn (24) des Stators (2) vorgesehen ist.

7. Rotationsmaschine nach einem der vorangehenden Ansprüche, wobei der Statorzahn (24) L-förmig ausgebildet ist und sich ein Schenkel des Statorzahns (24) parallel zur Rotationsachse (A) und sich ein anderer Schenkel des Statorzahns (24) radial zur Rotationsachse (A) zum Rotor (4) hin erstreckt.

8. Rotationsmaschine nach einem der vorangehenden Ansprüche, wobei der Rotor (4) eine unregelmässige Aussenkontur (41) mit einem radlal nach aussen gerichteten Rotorzahn (411) hat.

9. Rotationsmaschine nach einem der vorangehenden Ansprüche, wobei der Rotor (4) eine kreisförmige Aussenkontur (42) hat.

10. Rotationsmaschine nach einem der vorangehenden Ansprüche, wobei am Stator (2), im Speziellen am Polstück (5) eine Sensoreinrichtung (7) mit einem Sensor zur Bestimmung einer Magnetfeldstärke vorgesehen ist.

11. Rotationsmaschine nach Anspruch 10, wobei der Sensor ein Magnetfeldsensor, insbesondere Hallsensor oder magnetoresistiver Sensor, oder einen Wirbelstromsensor ist.

12. Rotationsmaschine nach Anspruch 10 oder 11, wobei die Sensoreinrichtung (7) insbesondere zur Detektion einer Winkellage des Rotors (4) durch ein Array (71) von Sensoren gebildet ist und dieses am Stator (2) angeordnet ist.

13. Scheibenförmiger Rotor für eine Rotationsmaschine (1) nach einem der vorangehenden Ansprüche, wobei eine axiale Höhe (H) des Rotors kleiner oder gleich einem halben Durchmesser (D) des Rotors ist, und der Rotor ein ringartiger Rotor mit einer um eine Rotationsachse (A) des Rotors vorgesehenen Ausnehmung ist, **dadurch gekennzeichnet, dass** der Rotor ein ferromagnetisches Material (FM) und keinen Permanentmagneten umfasst.

14. Vorrichtung, Insbesondere Wafer Bearbeitungsanlage, Bioreaktoranlage, Pumpe (P), Mischer oder eine andere Vorrichtung mit einer Rotationsmaschine (1) nach einem der Ansprüche 1 bis 12 und / oder einem Rotor (4) nach Anspruch 13.

## Claims

1. A rotational machine, designed as a bearing free motor, including a stator (2) designed as a bearing and drive stator having a stator winding (3) and a disc-shaped rotor (4) stored magnetically contact free within the stator (2), wherein an axial height (H) of the rotor (4) is smaller than or equal to a half diameter (D) of the rotor (4) and with the rotor (4) being passively stabilized by reluctance forces with regard to the stator (2) both against a displacement along a rotational axis (A) of the rotor (4) and also against a tilting from an equilibrium position (G), and with the stator (2) including a permanent magnet (6) for the generation of a homopolar magnetic flux (HΦ), **characterized in that** the rotor (4) is a ring-like rotor (4) rotatably arranged about a pole piece (5) of the stator (2) and **in that** the rotor (4) includes a ferromagnetic material (FM) and no permanent magnet.

2. A rotational machine in accordance with claim 1, wherein the permanent magnet (6) is the pole piece (5) or is provided at the pole piece (5) and is polarized along the rotational axis (A).

3. A rotational machine in accordance with claim 1 or claim 2, wherein the permanent magnet (6) is provided in a circumferential region (21) of the stator (2) and is polarized along the rotational axis (A).

4. A rotational machine in accordance with any one of the preceding claims, wherein the permanent magnet (6) is provided in a base region (22) of the stator (2) and is polarized in a radial direction orthogonal to the rotational axis (A).

5. A rotational machine in accordance with any one of the preceding claims, wherein the permanent magnet (6) is provided in a cover region (23) of the stator (2) and is polarized in a radial direction orthogonal to the rotational axis (A).

6. A rotational machine in accordance with any one of the preceding claims, wherein the permanent magnet (6) is provided in or at a stator tooth (24) of the stator (2).

7. A rotational machine in accordance with any one of the preceding claims, wherein the stator tooth (24) is designed L-shaped and a shank of the stator tooth (24) extends in parallel to the rotational axis (A) and a different shank of the stator tooth (24) extends radially to the rotational axis (A) towards the rotor (4).

8. A rotational machine in accordance with any one of the preceding claims, wherein the rotor (4) has an irregular outer contour (41) having a rotor tooth (411) directed radially outwardly.

9. A rotational machine in accordance with any one of the preceding claims, wherein the rotor (4) has a circular outer contour (42).

10. A rotational machine in accordance with any one of the preceding claims, wherein a sensor device (7) having a sensor for the determination of a magnetic field strength is provided at the stator (2), specifically at the pole piece (5).

11. A rotational machine in accordance with claim 10, wherein the sensor is a magnetic field sensor, in particular a Hall sensor, or a magneto resistive sensor, or an eddy current sensor.

12. A rotational machine in accordance with claim 10 or claim 11, wherein the sensor device (7) is formed by an array (71) of sensors, in particular for the detection of an angular position of the rotor (4), and with the array being arranged at the stator (2).

13. A disc-shaped rotor for a rotational machine (1) in accordance with any one of the preceding claims, wherein an axial height (H) of the rotor is smaller than or equal to a half diameter (D) of the rotor, and the rotor is a ring-like rotor having a recess provided about a rotational axis (A) of the rotor, **characterized in that** the rotor includes a ferromagnetic material (FM) and no permanent magnet.

14. An apparatus, in particular a wafer machining plant, a bioreactor plant, a pump (P), a mixer or a different apparatus having a rotational machine (1) in accordance with any one of the claims 1 to 12 and/or a rotor (4) in accordance with claim 13.

## Revendications

1. Machine rotative, conçue comme un moteur sans palier, comprenant un stator (2) conçu comme un stator de palier et un stator d'entraînement avec un enroulement de stator (3) et un rotor (4) en forme de disque monté magnétiquement sans contact à l'intérieur du stator (2), dans lequel une hauteur axiale (H) du rotor (4) est inférieure ou égale à un demi-diamètre (D) du rotor (4) et le rotor (4) est stabilisé passivement par rapport du stator (2) contre un déplacement le long d'un axe de rotation (A) du rotor (4) ainsi que contre un basculement à partir d'une position d'équilibre (G) par des forces de réluctance, et le stator (2) comprend un aimant permanent (6) pour générer un flux magnétique homopolaire (HΦ), **caractérisé en ce que** le rotor (4) est un rotor de façon annulaire (4) disposé de manière rotative autour d'un élément polaire (5) du stator (2) et que le rotor (4) comprend un matériau ferromagnétique (FM) et pas un aimant permanent.

2. Machine rotative selon la revendication 1, dans lequel l'aimant permanent (6) est l'élément polaire (5) ou est prévu à l'élément polaire (5) et ledit aimant est polarisé le long de l'axe de rotation (A).

3. Machine rotative selon la revendication 1 ou 2, dans lequel l'aimant permanent (6) est prévu dans une zone périphérique (21) du stator (2) et est polarisé le long de l'axe de rotation (A).

4. Machine rotative selon l'une des revendications précédentes, dans lequel l'aimant permanent (6) est prévu dans une zone inférieure (22) du stator (2) et est polarisé dans une direction radiale orthogonalement à l'axe de rotation (A).

5. Machine rotative selon l'une des revendications précédentes, dans lequel l'aimant permanent (6) est prévu dans une zone supérieure (23) du stator et est polarisé dans une direction radiale orthogonalement à l'axe de rotation (A).

6. Machine rotative selon l'une des revendications précédentes, dans lequel l'aimant permanent (6) est prévu dans ou à une dent de stator (24) du stator (2).

7. Machine rotative selon l'une des revendications précédentes, dans lequel la dent de stator (24) est en forme de L et une branche de la dent de stator (24) s'étend parallèlement à l'axe de rotation (A) et une autre branche de la dent de stator (24) s'étend radialement à l'axe de rotation (A) vers le rotor (4).

8. Machine rotative selon l'une des revendications précédentes, dans lequel le rotor (4) a un contour extérieur (41) irrégulier avec une dent de rotor (411) dirigée radialement vers l'extérieur.

9. Machine rotative selon l'une des revendications précédentes, dans lequel le rotor (4) a un contour extérieur (42) circulaire.

10. Machine rotative selon l'une des revendications précédentes, dans lequel un dispositif de capteur (7) avec un capteur pour déterminer une intensité du champ magnétique est prévu au stator (2), en particulier à l'élément polaire (5).

11. Machine rotative selon la revendication 10, dans lequel le capteur est un capteur de champ magnétique, en particulier un capteur à effet Hall ou un capteur magnétorésistive, ou un capteur à courants de Foucault.

12. Machine rotative selon la revendication 10 ou 11, dans lequel le dispositif de capteur (7) est formé par un réseau (71) de capteurs, en particulier pour détecter une position angulaire du rotor (4) et ledit réseau est disposé au stator (2).

13. Rotor en forme de disque pour une machine rotative (1) selon l'une des revendications précédentes, dans lequel une hauteur axiale (H) du rotor est inférieure ou égale à un demi-diamètre (D) du rotor (4) et le rotor est un rotor de façon annulaire avec un cavité prévu autour d'un axe de rotation (A) du rotor, **caractérisé en ce que** le rotor comprend un matériau ferromagnétique (FM) et pas un aimant permanent.

14. Dispositif, en particulier un installation de traitement de Wafer, une installation de bioréacteur, une pompe (P), un mélangeur ou un autre dispositif avec une machine rotative (1) selon l'une des revendications 1 à 12 et / ou un rotor (4) selon la revendication 13.
